# EUROPÄISCHE PATENTANMELDUNG

(11) **EP 1 136 546 A1**
(43) Veröffentlichungstag der Anmeldung: **26.09.2001**
(21) Anmeldenummer: 01102374.4
(22) Anmeldetag: 02.02.2001
(51) Int. Cl.: C12M 1/113

(54) **Vorrichtung und Verfahren zur Methanisierung von Substraten unterschiedlicher Konsistenz in einem kontinuierlich bzw. semikontinuierlich arbeitenden Gleitschicht-Fermenter**

(30) Priorität: 07.02.2000 DE 10005390
(71) Anmelder: BAW, Burdissa Agrodienstleistungen und Warenhandels GmbH, 02625 Bautzen/Niederkalna (DE); Hoffmann, Manfred, Prof. Dr., 91746 Weidenbach (DE); Rück, Werner, 91732 Merkendorf (DE)
(72) Erfinder: Miersch, Stefan, 02627 Radibor (DE); Krüger, Peter, 02694 Malschwitz (DE); Hoffmann, Manfred, Prof.Dr., 91746 Weidenbach (DE); Rück, Werner, 91732 Merkendorf (DE)
(74) Vertreter: Matschkur, Peter

(57) **Zusammenfassung**

Vorrichtung zur Methanisierung von Substraten unterschiedlicher Konsistenz in einem kontinuierlich bzw. semikontinuierlich arbeitenden formstabilen oder -flexiblen Gleitschicht-Fermenter, dadurch gekennzeichnet, dass im Einschleusungsteil bevorratbare, temperierbare und mischbare Biomassen/Substrate über eine geeignete Einschleusungsvorrichtung, Syphon, Presskolben, Mischerschnecke, Zinkenförderer etc. in einen waagrecht oder schräg liegenden Fermenter eingeleitet werden.

## Beschreibung

Zur Biogasgewinnung werden methanisierbare Biomassen (z. B. Gülle oder Klärschlämme) entweder in flüssiger Form (Nass-Vergärung) oder in halbfeuchter Form (Trocken-Vergärung) in Fermentern mikrobiologisch genutzt. Die im landwirtschaftlichen oder kommunalen Bereich favorisierte Nass-Vergärung von Gülle oder Klärschlamm ist vorwiegend auf deren betriebsinterne Verwertung abgestellt. Wegen der geringen Energiedichte z. B. der Gülle lohnt sich kein Transport, was im Regelfall über die Größe dieser Anlagen deren Wirtschaftlichkeit begrenzt. Zudem müssen ausbeutungswürdige Biomassen (z. B. Rasenschnitte, Gras, Maishäcksel, Bioabfall) erst in einen pumpfähigen Zustand gebracht, laufend homogenisiert und auch als Flüssigkeit auf den landwirtschaftlichen Nutzflächen verteilt werden. Die vorgestellte Erfindung bezieht sich vorzugsweise auf eine Trocken-Vergärung mit Trockensubstanzgehalten über 13 %.

Zur Trocknen Vergärung sind mehrere Verfahrensvarianten bekannt:
- diskontinuierliche Batch-Verfahren
- kontinuierliche bzw. simikontinuierliche Verfahren

Den kontinuierlichen bzw. simikontinuierlichen Trocken-Gär-Verfahren ist gemeinsam, dass der Substrattransport im Fermenter entweder über Schwerkraft (fassdown-Prinzip), bzw. mechanisch (z. B. Transportschnecke, Schubkolben oder Zinkenförderer) oder in geschobenen Spezialbehältern (Gärkanalverfahren) erfolgt.

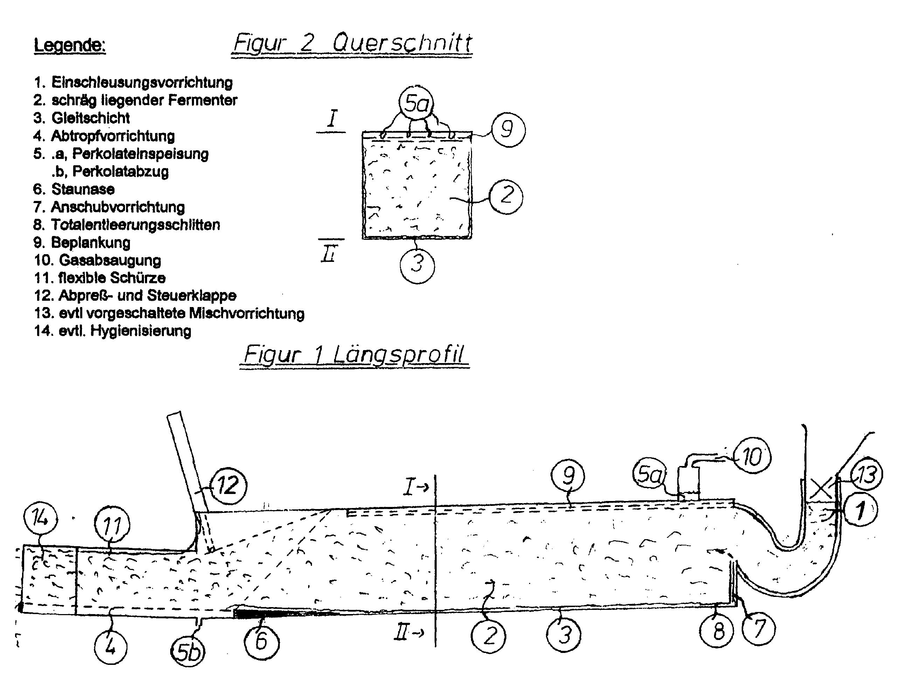

Bei der vorliegenden Erfindung handelt es sich um eine Vorrichtung zur wahlweisen Nass- oder Trocken-Vergärung in einem Gleitschicht-Fermenter, welcher im Längsprofil (Fig. 1) und Querschnitt (Fig. 2) vorgestellt wird. Dabei wird bezüglich der Fermenterbeschickung von einem wahlweise stufenlos oder absätzig arbeitenden einphasigen Vergärungsverfahren ausgegangen , welches mit einem Minimum an technischen Vorrichtungen auskommt.

Über eine Einschleusungsvorrichtung (z. B. Syphon, Presskolben, Mischerschneckej oder Zinkenförderer) wird das Gärgut kontinuierlich oder simikontinuierlich dem auf einer Geraden oder Schiefen Ebene angeordneten Gleitschicht-Fermenter zugeführt. Das Substrat gleitet dabei auf einer flüssigen Gleitschicht. wobei die "Fließgeschwindigkeit" vom Neigungswinkel des Fermenters, dem Substrat-Gewicht, insbesondere der Ausbildung und Qualität der Gleitflüssigkeit (Faulschlamm), der Reibung an den Fermenterwänden, vor allem aber der Einstellung einer mechanischen, pneumatischen oder hydraulischen Bremsvorrichtung im Ausschleusungsbereich abhängt.

Somit hat die Vorrichtung einen 3-teiligen Aufbau:
- eine Einschleusungsvorrichtung
- einen quaderförmigen oder röhrenförmigen formstabilen (Stahl- bzw. Stahlbetonelement) oder formvariablen (Kunststoffschlauch) modular erweiterbaren Fernenter-Teil
und
- einen Ausschleusungsteil.

Die Erfindung soll nachstehend anhand einiger Ausführungsbeispiele näher erläutet werden.

Beim Ausführungsbeispiel nach den Fig. 1 und 2 kann im (gasdichten) Einschleusungsteil auch eine Vortemperierung und Inoculation des Substrates erfolgen. Der sich langsam voranschiebende Masse-Strom gelangt in den Fermenterteil, in dessen vorderem Bereich sich eine Vorrichtung (z. B. ein mit Pressluft beaufschlagbares Luftkissen) zur evtl. erforderlich werdenden Überwindung der anfänglichen Haftreibung sowie eine Vorrichtung (z. B. Rechen) zur Totalentleerung befinden können, falls nicht eine entsprechende Einfüllvorrichtung für den entsprechenden Massenvorschub sorgt. Auf dem Fermenterboden bildet sich - vornehmlich durch die laufende Rezirkulation des entstehenden Perkolats- eine Gleitschicht, auf welcher das Substrat gleitend aufschwimmt. Der Fermenterteil schließt so ab, dass der Erhalt des Gleitfilms garantiert ist. Das Sammeln des Biogases und das Einbringen des Perkolats im oberen Fermenterbereich erfolgt über längsverlaufende und überlappend angeordnete Abdeckelemente, Rohre, Schläuche, etc. Der Ausschleusungsteil besteht aus einer luftdichten formlabilen Haube mit einer versteifbaren Rückwand, durch welche über einstellbare Pressruücke die Fließgeschwindigkeit des Substrat-Stromes reguliert werden kann, oder einem zweckdienlichen Formteil. Dabei kann die Ausschleusung soweit gedrosselt werden, dass auch fließfähiges Substrat verarbeitet werden kann. Gleichzeitig befindet sich im Ausschleusungsteil eine Abtropfvorrichtung (z. B. eine Rollenstrecke oder perforierter Boden) zum Sammeln des Perkolats, welches über einen Wärmetauscher temperiert rezirkuliert wird. Für eine allenfalls notwendig werdende Mischung von Faulsubstrat (Restgut) mit Biomasse (Frischsubstrat) befindet sich entweder im Ein- oder Ausschleusungsteil eine temperierbare Mischvorrichtung.

Eine andere Ausführungsvariante sieht eine Kammerung des Fermenterteils vor, wobei jede Kammer aus einem Substrat- und einem Gasspeicherteil besteht. Wird der Fermenter zur Zwecke des Entleerung zerstört (z. B. Kunststofffolie), so kann ein spezielles Ausschleusungsteil entfallen. In diesem Falle kann auch nach der Entgasung der Inhalt im Fermenter auch einer nachfolgenden Kompostierung unterzogen werden.

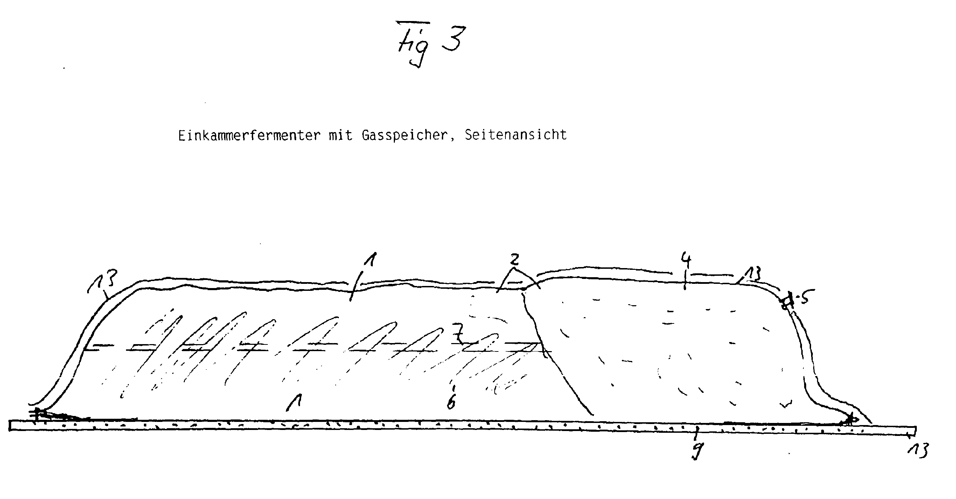

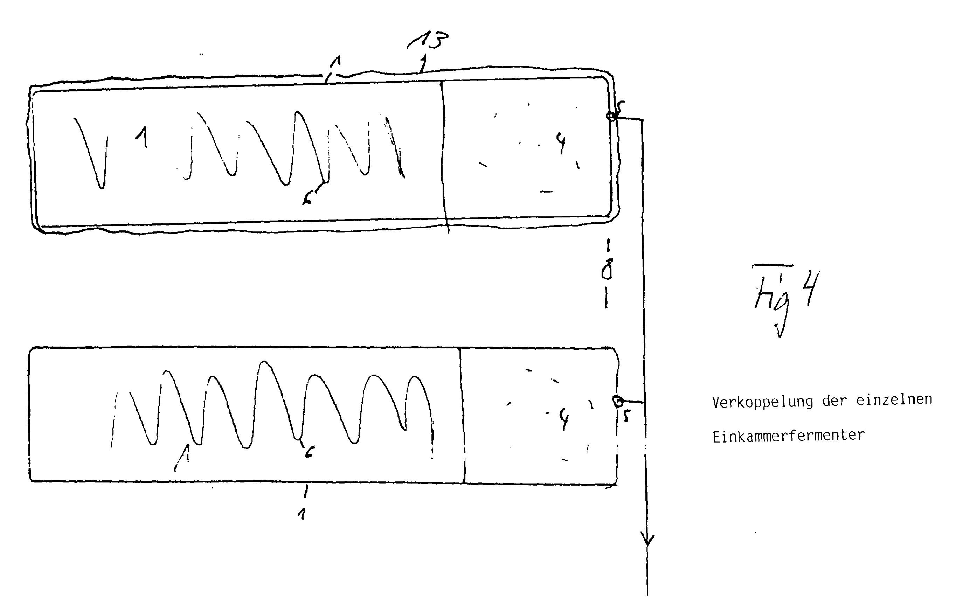

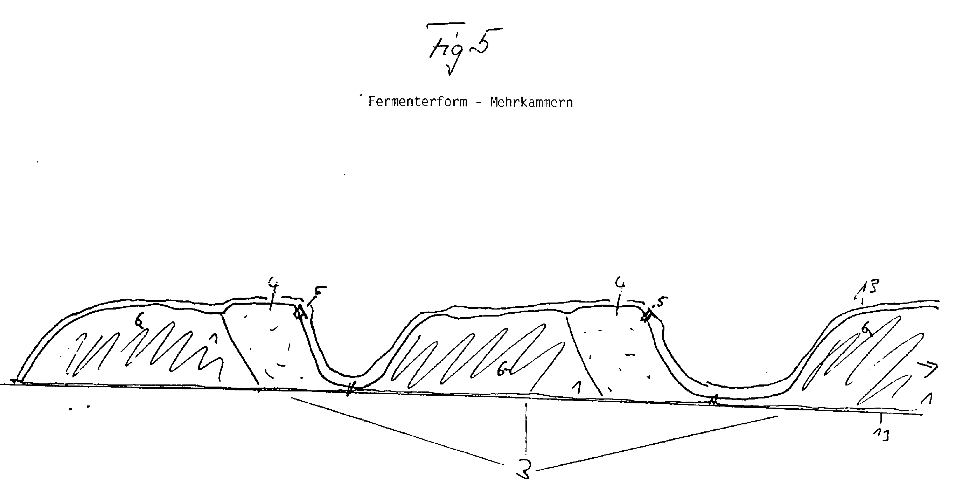

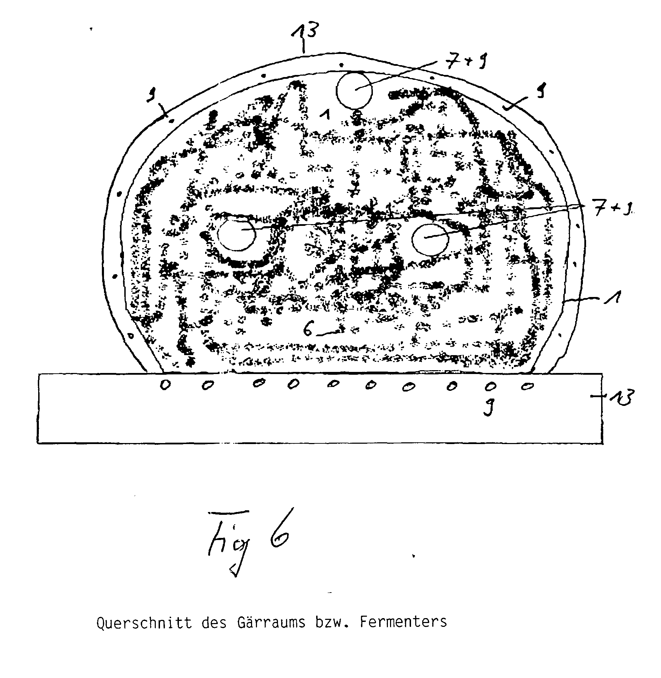

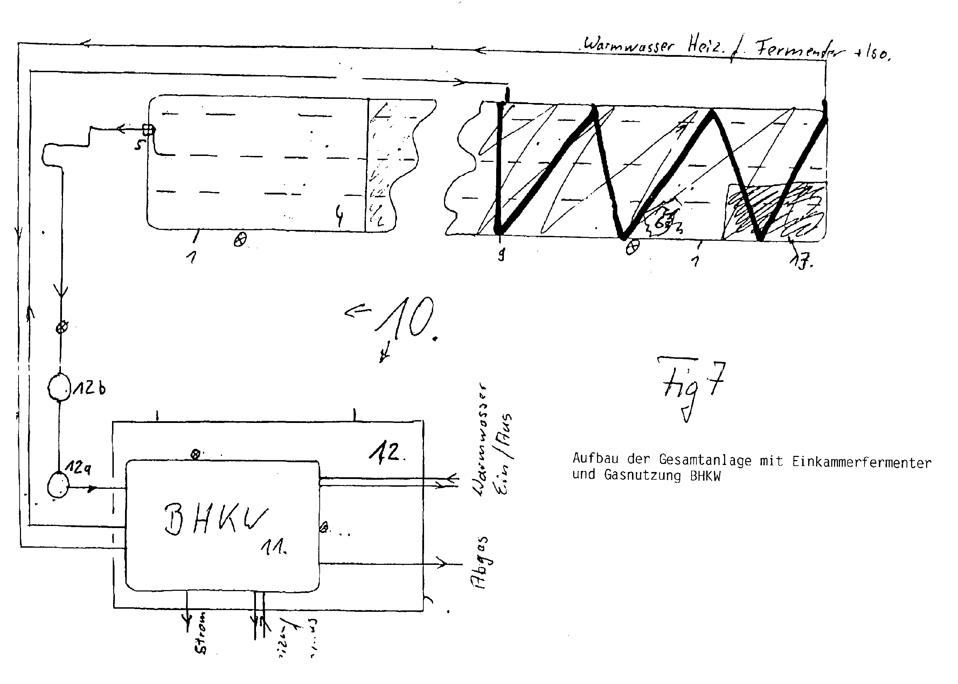

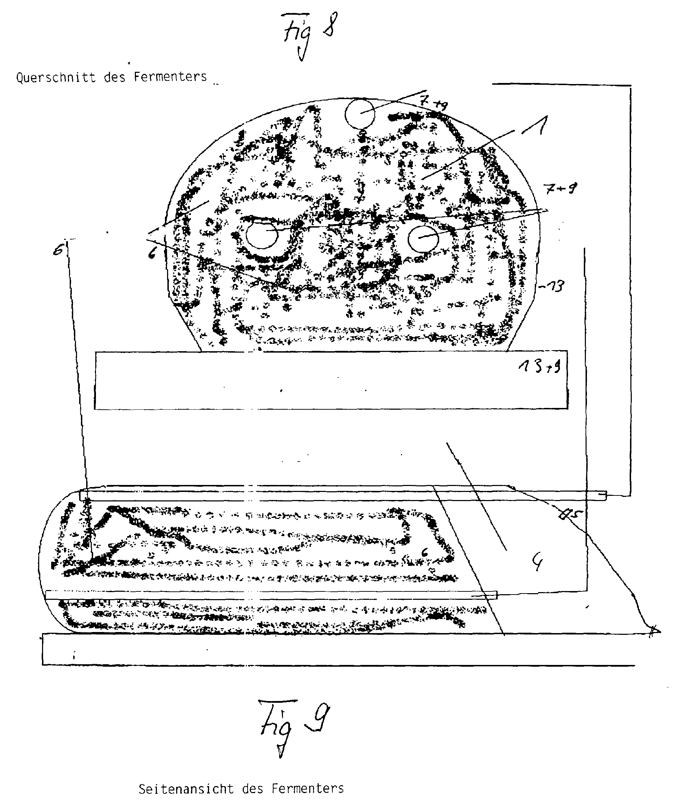

Eine andere Ausführungsvariante wie sie in den Figuren 3 bis 9 gezeigt ist, ist ein flexibler Folienschlauchfermenter bzw., Kunststofffermenter, im Sinne eines Gär. oder Fermenterraumes der in seiner Form, Größe und Art unterschiedlich sein kann. Der hier angesprochene Fermenter oder Gärraum fungiert als Ein- oder Mehrkammerfermenter, wobei jede Kammer aus einem Substrat- und einem Gasspeicher bestehen kann aber nicht zwingend muss. Wahlweise schließt sich an das Verfahren der Methanisierung von Substraten eine Hygenisierung auch durch eine mikrobielle Vorrotte des Substrates oder eine nachfolgende Kompostierung in der gleichen oder einer anderen Hülle an. Die Maschinen und Anlagen zur Befüllung jener Fermenter sind mobil, sie Verpressen das Substrat über vertikale Presskolben, Zinkenrotoren oder Forder- bzw. Transportschnecken Dabei ist man nicht vom bestimmten Substraten oder Zusammensetzungen jeglicher Art abhängig. Bei der Einlagerung findet eine Zerkleinerung, Durchmischung, Impfung - Applikation des Substrates statt. Der Gasabzug aus dem Fermenter bzw. Gasspeicher erfolgt über Spezialventile, die in die Fermenterhaut eingesetzt werden, diese sind mit der Gasleitung koppelbar. In die sich im Fermenter befindende Substratmasse können, falls notwendig, (bei der Kompostierung zwingend) Rohr zur Belüftung, Beheizung und zum Gasabzug eingebracht werden. Es besteht die Möglichkeit, die einzelnen Fermenter mit einander zu verbinden. Der Fermenter hat eine runde - ovale Form, er wird über seine Oberfläche, vom Kern oder wahlweise vom Boden beheizt. Die gesamte mobile aber auch stationäre Anlage umfass den eigentlichen Fermenter mit all seinen beschriebenen Merkmalen. Als auch eine Ansammlung von Maschinen und Geräten zur energetischen Nutzung des Methans, welche zum Gesamtpaket gehören, und aus diesem Grund auch in mobile Form d. h. im Container joder Wagen eingesetzt werden können. Dazu gehören: 12a) Methangaskühlung wasserumspült, 12b) Gasüber- bzw. Unterdrucksicherung auf Basis der Wasserverdrängung. Der Fermenter wird von allen Seiten isoliert, um Wärmeverluste zu minimieren (Isolierung 13).

## Patentansprüche

1. Vorrichtung zur Methanisierung von Substraten unterschiedlicher Konsistenz in einem kontinuierlich bzw. semikontinuierlich arbeitenden formstabilen oder -flexiblen Gleitschicht-Fermenter, **dadurch gekennzeichnet, dass** im Einschleusungsteil bevorratbare, temperierbare und mischbare Biomassen/Substrate über eine geeignete Einschleusungsvorrichtung, Syphon, Presskolben, Mischerschnecke, Zinkenförderer etc. (1) in einen waagrecht oder schräg liegenden Fermenter (2) eingeleitet werden.

2. Vorrichtung nach Anspruch 1, **dadurch gekennzeichnet, dass** sich auf dem Fermenterboden eine hydraulische Gleitschichtausbildung (3) entwickelt.

3. Vorrichtung nach Anspruch 1 und 2, **dadurch gekennzeichnet, dass** der aus den Baueinheiten "Einschleusung" - "Fermenterteil" - "Ausschleusung" bestehende Fermenter modular bzw. gekammert aufgebaut ist.

4. Vorrichtung nach einem der Ansprüche 1 bis 3, **dadurch gekennzeichnet, dass** eine Phasentrennung (4) im Ausschleusungsteil erfolgt.

5. Vorrichtung nach einem der Ansprüche 1 bis 4, **dadurch gekennzeichnet, dass** das temperierte Perkolat rezirkuliert wird.

6. Vorrichtung nach einem der Ansprüche 1 bis 5, **dadurch gekennzeichnet, dass** die hydraulische Gleitschicht (3) manipuliert (Staunase, Staurechen, etc. ) werden kann (6).

7. Vorrichtung nach einem der Ansprüche 1 bis 6, **dadurch gekennzeichnet, dass** der Fermenter einen formstabilen oder -flexiblen quaderförmigen, runden oder ovalen Querschnitt besitzt.

8. Vorrichtung nach einem der Ansprüche 1 bis 7, **dadurch gekennzeichnet, dass** eine hydraulische, pneumatische oder mechanische Vorrichtung (7) zur Überwindung von Haftreibung im Bedarfsfall eingesetzt und die durch eine Vorrichtung (8) zur Totalentleerung ergänzt werden kann.

9. Vorrichtung nach einem der Ansprüche 1 bis 8, **dadurch gekennzeichnet, dass** die Inoculation der Biomasse in der Einschleusungsvorrichtung vorwiegend durch Diffusion oder Mischung erfolgt.

10. Vorrichtung nach einem der Ansprüche 1 bis 9, **dadurch gekennzeichnet, dass** die Einspeisung für das Perkolat (5a) sich im oberen Fermenterteil (9), der Perkolatabzug (5b) sich im Ausschleusungsteil befinden.

11. Vorrichtung nach einem der Ansprüche 1 bis 10, **dadurch gekennzeichnet, dass** der Substratvorschub steuerbar ist.

12. Vorrichtung nach einem der Ansprüche 1 bis 11, **dadurch gekennzeichnet, dass** der Gasabzug (10) sowohl durch eigenen Gasdruck, als auch durch aktive Absaugung erfolgt.

13. Vorrichtung nach einem der Ansprüche 1 bis 12, **dadurch gekennzeichnet, dass** eine flexible Schürze (11) oder einem zweckdienlichen Formelement den Fermenterteil im Ausschleusungsteil abschließt.

14. Vorrichtung nach einem der Ansprüche 1 bis 13, **dadurch gekennzeichnet, dass** sich im Ein- oder Ausschleusungsteil eine beheizbare Mischvorrichtung (13) befinden kann.

15. Vorrichtung nach einem der Ansprüche 1 bis 14, **dadurch gekennzeichnet, dass** sich entweder im Einschleusungs- oder Ausschleusungsteil eine Hygenisierungsvorrichtung (14) befinden kann.

16. Vorrichtung nach einem der Ansprüche 1 bis 15, **dadurch gekennzeichnet, dass** die Hygenisierung auch durch eine mikrobielle Vorrotte des Substrates oder eine nachfolgende Kompostierung erfolgen kann.

17. Vorrichtung nach einem der Ansprüche 1 bis 16, **dadurch gekennzeichnet, dass** die Ent- und Versorgung statt mit Rohren oder anderweitigen Vorrichtungen auch mit fallweise einführbaren Einstechzylindern erfolgen kann.

18. Vorrichtung nach einem der Ansprüche 1 bis 17, **dadurch gekennzeichnet, dass** eine Isolation in die Fermenterwandungen integriert ist.

19. Vorrichtung nach einem der Ansprüche 1 bis 18, **dadurch gekennzeichnet, dass** die Maschinen oder Anlagen zur Befüllung jener Folienbehältnisse mobil sind und sich in der Regel aller Fälle gegen einen frei zu bestimmenden Bremsdruck vom Folienbehältnis wegbewegen - Fahrtrichtung.

20. Vorrichtung nach einem der Ansprüche 1 bis 19, **dadurch gekennzeichnet, dass** bei der Einlagerung des Substrates eine Zerkleinerung, Durchmischung, Impfung (-Gülle-) des selben stattfindet, ohne beim Bau bzw., Einsatz der Maschinen oder Anlagen einen Mehraufwand zu betreiben, wobei ggf. bei dem Prozess der Einlagerung das Ausgangssubstrat mit Organismen beimpft wird.

21. Vorrichtung nach einem der Ansprüche 1 bis 20, **dadurch gekennzeichnet, dass** der Gasabzug aus dem Folienbehälter über Spezialventile, welche in die Fermenterhaut am Ort der Gasentnahme eingesetzt werden, oder aber auch durch Einschlag- oder Einschraubzylinder, welche in das Substrat gesteckt, gesetzt oder geschlagen werden und später auch für die Zwecke der Kompostierung genutzt werden können, erfolgt.
